(19) Europäisches Patentamt
European Patent Office
Office européen des brevets

(11) Publication number: **0 585 018 A1**

# (12) EUROPEAN PATENT APPLICATION

(21) Application number: **93306417.2**

(22) Date of filing: **13.08.93**

(51) Int. Cl.$^5$: **A61K 7/42,** A61K 7/13

(30) Priority: **17.08.92 GB 9217444**

(43) Date of publication of application:
**02.03.94 Bulletin 94/09**

(84) Designated Contracting States:
**AT BE CH DE DK ES FR GB GR IE IT LI NL PT SE**

(71) Applicant: **UNILEVER PLC**
**Unilever House Blackfriars**
**London EC4P 4BQ (GB)**
(84) **GB IE**

(71) Applicant: **UNILEVER N.V.**
**Weena 455**
**NL-3013 AL Rotterdam (NL)**
(84) **BE CH DE DK ES FR GR IT LI NL PT SE AT**

(72) Inventor: **Cheetham, Pèter Samuel James**
**28 Meadway**
**Harrold, Bedfordshire MK43 7DR (GB)**
Inventor: **Lee, Caroline Marian**
**11 Crayton Road**
**Ampthill, Bedfordshire MK45 2LE (GB)**
Inventor: **de Graaf, Thalie Paulina**
**63 Greenshields**
**Bedfordshire MK40 3TU (GB)**

(74) Representative: **Ford, Michael Frederick et al**
**MEWBURN ELLIS 2 Cursitor Street**
**London EC4A 1BQ (GB)**

## (54) Cosmetic composition for coloring hair and skin and for UV-protection.

(57) A composition for colouring the hair or skin containing as active ingredient the product of the reaction between an alkyl ester of the formula:

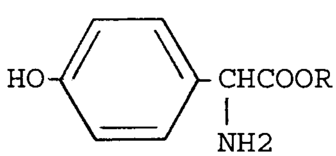

wherein R = $C_{1-6}$ alkyl, tyrosinase and optionally cysteine. The composition additionally offers sun shielding against UVA and UVB radiation. The active ingredient is able to bind to skin or hair protein so enhancing the longevity of the colour produced.

EP 0 585 018 A1

Field of the Invention

The present invention is concerned with compounds, compositions and methods for producing or restoring colour in hair or skin; the invention is also applicable to sun screening. It finds particular application in topical compositions for the restoration of colour to hair and the colouring of skin, as well as in sun shielding.

Summary of the Prior Art

In the prior art, US-A-4021538 discloses the application of various esters of dopa (3,4-dihydroxyphenylalanine) to the skin and in particular L-methyl dopa is stated to penetrate the skin and to cause in many cases immediate pigmentation. No mechanism is given for this action. Dopa is an active drug used inter alia for the treatment of Parkinson's Disease.

In US-A-4515773 there is a discussion of the use of tyrosinase and a pigment precursor such as L-tyrosine, L-dopa, D-dopa or "other related melanin precursors or analogs" which are unspecified. The action of the tyrosinase is stated to be to catalyse the formation of the melanin-like pigment on the surface of the skin. In J Soc. Cosm. Chem. 40, 65 (1989) there is disclosure that 5, 6-dihydroxyindole may be used to colour hair.

EP-A-318369 discloses the use of tyrosine or derivatives thereof including tyrosine to enhance sun-tanning, that is to say to enhance the extent to which melanin is formed in skin when exposed to ultra-violet light.

In Helv Chim Acta 45, 638 (1962) and 51, 1476 (1968) investigations of the synthesis of UV-absorbing material from L-methyl-dopa are described.

Existing pigmentation products, for instance using dihydroxyacetone, see EP-A-456545, often produce an uneven streaky appearance and a poor shade of colour. Also, the colour-forming active ingredients in existing tan enhancers do not provide protection against UVA and UVB radiation.

Summary of the Invention

The present invention is based on the discovery that if a lower alkyl ($C_{1-6}$) tyrosine ester of the formula

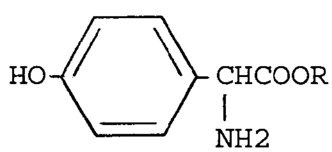

wherein R is a $C_1$-$C_6$ alkyl, preferably methyl or ethyl, are incubated with tyrosine, previously undescribed oligomers and polymers of the tyrosine ester are formed. These products are extremely valuable pigments and also combine UVA and UVB sun shielding effects.

In one aspect, this invention provides a composition for application to skin or hair, containing as active ingredient the product of reaction between

(i) an alkyl tyrosine ester of the formula:

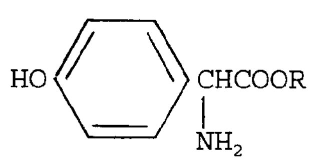

where R = $C_{1-6}$ alkyl, and

(ii) tyrosinase.

The invention also provides a composition containing a said alkyl tyrosine ester and tyrosinase.

The ester and the tyrosinase may be applied directly to skin or hair, in either order, so that the reaction which is brought about by tyrosinase takes place on the skin or the hair. The invention therefore also provides a kit which includes at least the alkyl tyrosine ester and the tyrosinase in separate containers for simultaneous or subsequent application to the skin or hair.

The invention provides also a method of pigmenting the hair or skin, with the additional benefit of providing a sun screen for the hair or skin. This consists of applying to the hair or the skin a system consisting of an alkyl tyrosine ester and tyrosinase, or a product formed by the reaction of these components.

## Brief Description of the Drawings

Figure 1 is a graph showing the absorbence of chromophores obtained from methyl and ethyl tyrosine esters and comparing with tyrosine as precursor compound.

Figure 2 is a graphical comparison of the absorptivity of the present chromophore with those of two known sunscreening preparations.

Figure 3 shows graphically the effect of ageing on the chromophore.

Figure 4 shows the effect on absorptivity of conjugation of the present chromophore with casein and keratin respectively.

Figure 5 shows graphically the effect of storing the chromophore in glycerol.

Figure 6 shows the UV spectrum of the product obtained in the presence of cysteine.

Figure 7 shows the effect on absorbence at 330 nm of freezing and drying a solution containing the chromophore produced in the reaction between methyl tyrosine ester, tyrosinase and cysteine.

## Detailed Description and Embodiments

Tyrosinase is a bifunctional enzyme which in man occurs in the melanosomes in the basal layers of the epidermis. Its function is to catalyse the meta-hydroxylation of tyrosine to yield L-dihydroxyphenylaline (L-DOPA) and then to catalyse the oxidation of L-DOPA to a quinone, L-DOPA quinone.

For this invention, tyrosinase may be derived from sugar beet, which provides a cheap and abundant source. The tyrosinase may be immobilized on a carrier if desired. Mushroom tyrosinase may also be used; it is available commercially from Sigma.

We believe that there has been no previous disclosure of UV-absorbing compounds formed by the reaction of an alkyl ester of tyrosine brought about by tyrosinase.

As mentioned above, we have found that a valuable chromophore is produced by this enzymic reaction. The reaction can be carried out in vitro, in aqueous solution, leading to a coloured solution of the chromophore. By contrast tyrosine itself does not lead to such a chromophore.

Without limitation to any theory, we believe that the action of tyrosinase on alkyl esters of tyrosine produces such products as the isomeric compounds

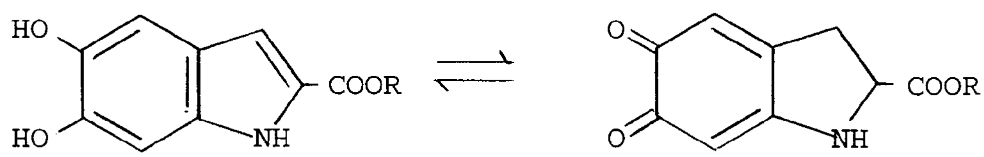

wherein R is $C_{1-6}$ alkyl, preferably methyl.

Oligomers and polymers of these compounds are absorbers of both ultraviolet A (radiation of about 320 to 360 nm) and ultraviolet B (radiation of about 280 to 320 nm), having a maximum absorbence in the region of 330 nm. Hence they are extremely valuable compounds for use not only in colouring or colour-restoring compositions but also in sun-shielding compositions since they exert both a radiation-shielding and a colouring effect.

The formation of an advantageous chromophore by means of the reaction of a tyrosine ester and tyrosinase is illustrated by Figure 1 which is a graph of the absorbences of chromophores formed by the reaction of respectively (1) methyl tyrosine, (2) ethyl tyrosine and (3) tyrosine itself, with tyrosinase, in the ultraviolet region (approx 280 to 360 nm).

It can be seen that the absorbence of the tyrosine ester derivatives peaks in the region of 320 and 330 nm, an area of particular interest since it is midway between the ultraviolet A and ultraviolet B ranges and in most sun screening compositions these two ranges have to be dealt with by distinct components.

In contrast the derivative from tyrosine showed no significant effectiveness at this or any other relevant wavelength.

Figure 2 offers comparison between the methyl tyrosine derivative and two known sunscreen protective agents namely Parsol MCX and Parsol 1789 which are in widespread commercial use. Although the absorbency peaks of both of these preparations are higher than that of the methyl tyrosine derivative, they lie respectively in the ultraviolet A and B regions, while the peak for the methyl tyrosine derivative lies between those regions and there is ultraviolet absorption on both sides of that peak.

A chromophore as produced in aqueous solution in accordance with the present invention has reasonable

stability in solution over a short term. Over a period of time it undergoes slow change as illustrated by Figure 3 which shows the effect of ageing of the product from methyl tyrosine and tyrosinase. The solid line shows that after 18.5 hours the absorbency is hardly changed from that of the freshly prepared product although there is a slight sharpening of the peak. After two weeks the absorbency shows substantially no peak (although there is a slight inversion at around 310 nm), but absorbence is higher at the shorter wavelengths, i.e. in the ultraviolet B region. After a long period the profile of the curve remains unchanged, although the total absorbency is lower due to the precipitation of product.

This indicates that the present chromophores may retain useful sunscreening utility after a considerable period on the skin.

The aqueous solution of chromophore, produced by reaction of a tyrosine ester and tyrosinase cannot be dried without special measures to protect it. If this is attempted the chromophore polymerises as the solution becomes concentrated and an insoluble precipitate is formed.

In view of this the invention can be utilised in various ways. One possibility is to stabilise the chromophore against polymerisation (which will be discussed further below). Another possibility is to carry out the reaction of the ester and tyrosinase shortly before application of the chromophore to the skin or hair, and yet another possibility is to carry out the reaction in situ on the skin or hair.

Reaction to form the oligomers and subsequent polymers in situ on the skin or hair will take place once the tyrosine ester and tyrosinase are both present.

If the composition is to be applied with the components at that time unreacted, they may be applied from a kit with the tyrosine ester and the tyrosine in separate containers.

Another possibility is to apply them as a single composition in which the constituents are prevented from reacting with each other until rubbed on the skin, such as by separate microencapsulation.

Both the oligomers and the polymers have the advantageous property that if their components react in situ on skin they bind strongly to skin proteins, e.g. keratin, some reaction occurring within the skin itself in the basal layer of the epidermis. Such colouring resists abrasion and washing.

Figure 4 shows the modification of ultraviolet absorption when the chromophore produced from methyl tyrosine is conjugated to casein and to keratin. Although there is some loss of absorbency in the conjugated derivative and some shift of the peak towards the ultraviolet A region, neither the loss nor the shift is serious. This conjugation is of particular importance since it both offers a mode of increasing the substantivity of the tan and provides UVA and UVB protection by the binding of the chromophore to the skin. Substantivity is of great importance from the point of view of avoidance of loss of protective effect due to abrasion, washing and swimming, a serious problem with most sunscreening preparations.

If the reaction of tyrosine ester and tyrosinase takes place in situ on the hair, strong binding will again occur.

If it is not desired that this strong binding should occur, solubilized proteins such as casein or, especially, hydrolysed keratin can be included in the initial reaction mixture which produces the oligomers. The chromophore produced in the reaction can then couple to the protein and is thereby stabilized.

A further possibility for stabilizing the chromophore is to add a polymerisation inhibitor effective against the polymerisation of quinones, such as sodium metabisulphite or glutamic acid.

One possibility for stabilising the chromophore when it is produced in aqueous solution is to carry out the reaction in the presence of a protein such as casein or hydrolysed keratin. The chromophore binds to the protein and we have found that it is then much more stable.

Another possibility for stabilising the chromophore when it is produced in aqueous solution is to add glycerol or ethylene glycol (both of which are hydrophilic organic solvents) and then remove the water so as to concentrate the chromophore into this organic solvent. We have found that the resulting concentrate can later be diluted with water to an aqueous solution, with substantial retention of the UV absorbency.

Figure 5 illustrates that the product derived from methyl tyrosine and tyrosinase can be manipulated in this way without serious loss of its absorptive effects. Line (a) on the graph shows the absorption spectrum of the original chromophore in glycerol. Line (b) shows the spectrum after the derivative had been concentrated into glycerol, had been stored and then had been redissolved to the original concentration in water.

Another possibility for stabilising the chromophore produced in aqueous solution is to absorb the oligomeric product of reaction onto a solid absorbent. Possibilities are ion exchange resin such as Amberlite XAD, charcoal, talc, silica or titanium dioxide.

Oligomer sorbed onto some of these solids, for example, talc, silica or titanium dioxide can be utilised directly in some product forms.

A $C_1$ to $C_6$ alkyl ester of tyrosine may be used as the sole pigment forming material. However, in a significant development of this invention the pigment-forming system comprising a tyrosine ester and tyrosinase is supplemented by including cysteine. This is advantageous in that the colour of the resulting pigmentation is more pleasing, being of a reddish bronze colour, than that resulting from the reaction of alkyl tyrosine ester and tyrosinase alone, which is of a dull brown colour. The chromophore formed is also more soluble and less prone to polymerisation, leading to the formation of insoluble precipitates.

This development of the invention also provides new compositions of matter. In particular it provides a product of the reaction of alkyl tyrosine ester and cysteine, namely 5-cystinyl alkyl dopa of the formula I and benzothiazinyl alkyl alanine of the formula II formed by ring-closure in the same medium of the compound of formula I, which are both novel compounds:

I

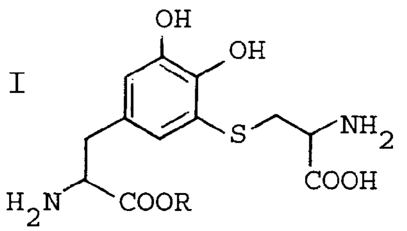

II

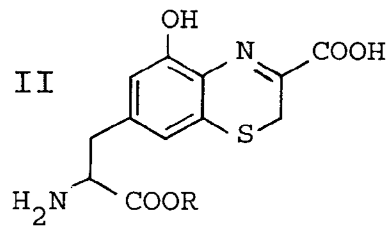

Other possible products formed by the cyclisation of a further side chain of I or II are:

III

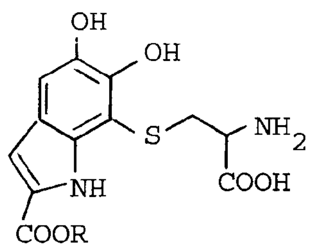

IV

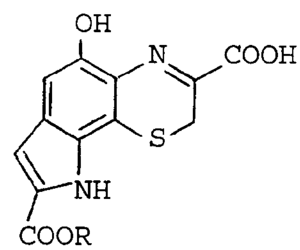

wherein R is $C_{1-6}$ alkyl - preferably methyl.

I, II and III are in equilibrium with the corresponding diketo (quinone) forms.

The formation of the compound I occurs in aqueous media at a wide variety of pH values in the presence of tyrosinase, which is the cause of the substitution of the second hydroxyl on the phenyl ring. If the cysteine were not present, polymerisation to an insoluble dark brown polymer would be more rapid. However, the cysteine is believed to form an intermediate that is less prone to polymerisation.

Condensation of the compound of formula II yields alkyl pheomelanin as soluble oligomers and insoluble polymers both of which are pigmented UV absorbers, which are also believed to be new per se.

Figure 6 shows the ultraviolet absorbency of a chromophore produced from methyl tyrosine, cysteine and tyrosinase. The spectrum was taken when the chromophore-producing reaction had reached completion. It can be seen that the ultraviolet absorption is very similar to that in Figure 1, with a strong peak at 320 nm.

We have found that when cysteine is present the chromophore is more soluble in water and also more stable against unwanted polymerisation. When cysteine is incorporated in the preferred proportion relative to tyrosine ester, the solution of chromophore could undergo freeze drying and rehydration with retention of much of the ultraviolet absorption. This is illustrated by Figure 7 which shows the absorption at 330 nm for reaction solutions produced from methyltyrosine (4mg per 100 ml) and various quantities of cysteine.

The upper line shows absorbency of the reaction mixture. It shows that this falls off when the weight of cysteine exceeds the weight of methyltyrosine. The lower plot shows the absorbency after freeze drying and rehydration. At a 1.6:1 weight ratio of tyrosine ester to cysteine, the absorbency is very largely preserved through drying and recovery.

The reaction of tyrosine ester in situ on the skin can take place using endogenous tyrosine. Consequently, it would be possible to provide both tyrosine ester and cysteine in a composition for topical applications to make use of endogenous tyrosinase. This therefore is a further aspect of this invention.

Compositions in accordance with this invention will generally contain the chromophore produced by reaction, or alternatively tyrosine ester, tyrosinase and optionally cysteine, in a cosmetically acceptable carrier vehicle.

A wide variety of such vehicles are known. At the simplest the vehicle may be water, but it will frequently include liquids or solids which perform a function, such as emollients, humectants and thickeners. Other active components (such as conventional sunscreens, penetration aids) and additives (such as perfumes, stabilizers and other colourants) may be present.

A composition may be formulated in such products forms as a mobile lotion, a thickened emulsion and a stiff emulsion like an ointment. Emulsions may be water-in-oil or oil-in-water types.

A composition of this invention will generally contain chromophore oligomer at a concentration from 0.01 to 10%, preferably 0.1 to 5% by weight. To achieve such concentrations, water may be removed from a reaction solution by freeze drying, utilising means to stabilise the oligomer as described above. Stabilisation during concentration may not be necessary if cysteine was present in the reaction mixture.

For preparation of the oligomers, the weight ratio of tyrosine ester to tyrosinase will generally lie in a range from 100:1 to 1:1.

A composition containing tyrosine ester e.g. part of a kit for use in effecting reaction in situ on skin, may contain the ester in amounts from 0.001 to 0.1% by weight or possibly more up to 5% by weight although this is not preferred.

## EXAMPLE 1

### Reaction of methyl (or ethyl) tyrosine ester with tyrosinase

0.002g of methyl (or ethyl) tyrosine ester available from Sigma Chemical was dissolved in 100g of water. The pH was then adjusted to pH 7.0 after which 0.00033g tyrosinase (Sigma) was added and the reaction allowed to proceed at 20-30°C. The formation of the UV-absorbing reaction products was monitored using a Shimadzu double beam spectrophotometer (type UV-2101PC) using as a reference an aqueous solution containing 0.02$gl^{-1}$ of methyl tyrosine.

Initial formation of colour was rapid, but the UV absorbency continued to increase over some hours. The resulting UV spectra are shown in Figure 1 as described above. When the reaction was carried out with tyrosine in place of one of its esters the product was not effective as a chromophore.

It was found that the aqueous solutions of chromophore obtained in this way suffered slow polymerisation during storage.

During storage at room temperature or 4°C for two weeks under an air atmosphere, both in light and dark conditions, the ultraviolet spectrum changed to some extent but absorption at about 330nm did not change very much. This is illustrated by Figure 3 above.

The ultraviolet absorption at around 330nm also remained stable at 40°C for one week at pH values as low as 2 and up to at least 9. No effect was seen under prolonged oxygenation or when subjected to microbial or plant esterases. Bleaching had little effect on the ultraviolet spectrum absorptivity but removed a lower peak which occurs at around 215 nm.

The chromophore produced as described above had an absorbency at 310 nm at a concentration of 1% w/v in a 1 cm cell of greater than 400, i.e. $E^{1\%}_{310}$ = 400.

If instead of carrying out the process as described, fresh enzyme is added after 24h, and is incubated with the methyl or ethyl tyrosine, a higher peak with an $E^{1\%}_{310}$ value of approximately 700 is obtained.

Higher concentrations of chromophore can be achieved by sequential additions of portions of precursor and/or enzyme. However, use of excess concentrations of precursor at the beginning of the reaction was not very effective in increasing chromophore concentration.

## EXAMPLE 2

### Reaction of methyl tyrosine ester with tyrosinase and cysteine

Reaction generally as in Example 1 was modified by the initial inclusion of cysteine. Reactions were carried out using 0.04$gl^{-1}$ of methyl tyrosine and from 0.01 to 0.12$gl^{-1}$ of cysteine which were warmed to dissolve the reactants, cooled, and then 3.5$mg1^{-1}$ of tyrosinase added. Best results were obtained using a cysteine concentration of 0.02-0.03$g1^{-1}$ and a methyl tyrosine to cysteine weight ratio of 1.33:1 to 2.0:1.

The rate of the reaction was monitored by observing UV absorption at 320 nm. The rate was found to be inversely proportional to the concentration of cysteine. At optimum concentration, completion was reached in six to seven hours. The intensity of the UV absorption spectrum also decreased as the amount of cysteine was increased, although the intensity of the final visible colour was not affected.

The chromophore produced has similar UV absorption properties to that produced by methyl tyrosine alone with tyrosinase, as illustrated by Figure 6. but in the visible spectrum has a more attractive appearance being of a reddish/bronze hue.

EXAMPLE 3

Stabilization of the chromophore in vitro.

The reaction of methyl tyrosine and tyrosinase as described in Example 1 was carried out in the presence of casein or partially hydrolysed keratin at concentrations of at least 1g per litre of reaction mixture.

The UV absorption was modified, but not seriously harmed, as illustrated by Figure 4.

Following the reaction the UV chromophore was no longer dialysable, indicating that the molecular weight of the product had been greatly increased through coupling to the keratin or casein.

The reaction was repeated using a sample of unpigmented human hair instead of the keratin solution. After the reaction was complete, the hair became darker in colour and UV absorption of the solution was significantly lowered relative to a control solution of methyl tyrosine and tyrosinase alone. The hair colour produced using methyl tyrosine was resistant to washing whereas the colour formed in a control experiment using tyrosine was easily removed from hair.

A solution as produced in Example 1 did not survive freeze drying. It was observed that polymerisation and formations of a precipitate occurred as the solution was concentrated.

By contract, when keratin was included in the reaction mixture at concentrations ranging from 1 to 6 grams per litre, the product could be dried and later rehydrated with retention of substantially all of its UVA and UVB absorbency.

EXAMPLE 4

Stabilization of the chromophore

The UVA and UVB chromophore was stabilized against concentration or drying by:

(i) the addition of 0.1 ml glycerol to 100 ml of the reaction solution of Example 1 (i.e. 50ml of glycerol per gram of methyl tyrosine) or

(ii) the addition of 0.025g of sodium metabisulphite to 100 ml of reaction solution (i.e. 12.5 gram per gram of methyl tyrosine) or

(iii) the addition of 0.01g of glutamic acid to 100ml of reaction solution (i.e. 5 gram per gram of methyl tyrosine).

The stabilized product was then freeze-dried in a Virtis freeze dryer producing a brown solution from (i) and a brown powder from (ii) and (iii).

It was found that the solution or powders can be stored indefinitely under ambient conditions and then redissolved in water to give solutions having substantially the same UV spectrum and colour characteristics of the original solution prior to stabilization. The results with glutamic acid were improved by using 0.1g or more per 100ml of solution. The good results with glycerol and metabisulphite were maintained at higher concentrations of these materials.

EXAMPLE 5

Reaction of the Products with excised human skin

Methyl tyrosine ($2gl^{-1}$) and tyrosinase ($0.03gl^{-1}$) were allowed to react at pH7 and 20°C for at least 2 hours. This solution containing the product was then layered onto the surface of isolated human skin and left for 2 hours at 20°C for reaction to take place. The skin was then frozen and cryostat sectioned, and compared with skin treated with a saline control.

Examination of the treated skin samples by light microscopy showed that the addition of the product solution had produced a brown colour in the outer layers of the stratum corneum. The pigmentation had penetrated 2 to 3 cell layers deep. No pigmentation was present in the saline control sample.

In a second experiment, only the methyl tyrosine solution was added to skin samples. In this case a brown pigmentation developed which was not only restricted to areas at the basal layer of the epidermis where endogenous melanocyte tyrosinase should be located, but also throughout the basal layer. Once again no pigmentation was observed in the control.

EXAMPLE 6

A sunscreen composition is formulated with methyl tyrosine ester and tyrosinase as follows:-

Methyl tyrosine ester and tyrosinase are separately microencapsulated by a standard procedure. A composition is made up containing the methyl tyrosine ester microcapsules and the tyrosinase capsules in an aqueous medium, together with surfactants, perfume, colourant and other additives.

On spreading on the skin the microcapsules of the active components are broken and the reaction outlined above occurs, with binding of the chromophore produced to the skin proteins such as keratin. After prolonged exposure the colour of the skin darkens further, which is taken to be a result of further polymerisation of the chromophore with the absorption shift indicated in Figure 3.

EXAMPLE 7

A chromophore oligomer is prepared as in Example 2 and is formulated into an oil continuous cream as follows:

| Ingredients | %w/w |
|---|---|
| Silicone oils | 24.00 |
| Whitener | 0.15 |
| Humectants | 5.00 |
| Chromophore oligomer | 1.00 |
| Lactic acid | 5.00 |
| Potassium hydroxide | 4.00 |
| Water | 60.85 |
| | 100.00 |

The skin cream, having a pH value of 5, is made by adding gradually to a mixture of silicones and whitener an aqueous mixture of the remaining ingredients and homogenising.

EXAMPLE 8

This example illustrates a water-continuous (i.e. water-in-oil) cream containing the chromophore prepared in Example 2.

| Ingredients | %w/w |
|---|---|
| Thickener | 0.50 |
| Whitener | 0.20 |
| Humectant | 10.00 |
| Evening Primrose Oil | 2.00 |
| Emulsifiers | 10.50 |
| Silicone Oil | 7.60 |
| Chromophore oligomer | 3.00 |
| Triethanolamine | 6.00 |
| Lactic Acid | 4.00 |
| Water | 56.20 |
| | 100.00 |

EXAMPLE 9

A chromophore oligomer is prepared as in Example 2 and is formulated into a solution used to condition hair.

| Ingredients | %w/w |
|---|---|
| Emulsifier | 0.80 |
| Chromophore oligomer | 0.50 |
| Sodium chloride | 0.50 |
| Sodium hydroxide | 2.00 |
| Water | 96.20 |
| | 100.00 |

EXAMPLE 10

A chromophore prepared as in Example 2 is stabilized by being absorbed onto titanium oxide particles. A lotion is prepared to the following formulation:

| Ingredients | %w/w |
|---|---|
| Silicone surfactant (Dow Corning DC 3225C) | 10.00 |
| Volatile siloxane (Dow Corning DC 345) | 14.00 |
| Mineral Oil | 1.50 |
| Ultrafine titanium dioxide (water-dispersible) | 5.00 |
| 2-hydroxy octanoic acid | 1.00 |
| Lactic acid | 5.00 |
| Butylene glycol | 10.00 |
| Sodium chloride | 2.00 |
| Chromophore oligomer | 1.00 |
| Sodium hydroxide | 5.00 |
| Perfume | qs |
| Water | Balance to 100% |

## Claims

1. A composition for application to skin or hair to serve as a colourant and/or sunscreen therefor containing as active ingredient the product of the reaction between:
   (i) an alkyl tyrosine ester of the formula:

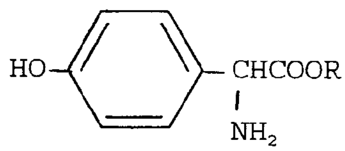

   where R = $C_{1-6}$ alkyl,
   (ii) tyrosinase, and optionally
   (iii) cysteine

2. A composition according to claim 1 further comprising protein with the said active ingredient bound thereto.

3. A composition according to claim 1 wherein the composition comprises polymerisation inhibitor for the active ingredient.

4. A composition according to claim 3 wherein the polymerisation inhibitor is selected from the group consisting of sodium metabisulphite and glutamic acid.

5. A composition according to claim 1 which includes a hydrophilic organic solvent.

6. A composition according to claim 5 wherein the solvent is glycerol.

7. A composition according to claim 1 wherein the active ingredient is the product of reaction of a said alkyl tyrosine ester, cysteine and tyrosinase, with the tyrosine ester and cysteine in a weight ratio in the range 1.33:1 and 2.0:1.

8. A composition according to claim 1 wherein the active ingredient comprises a compound selected from the group consisting of

I 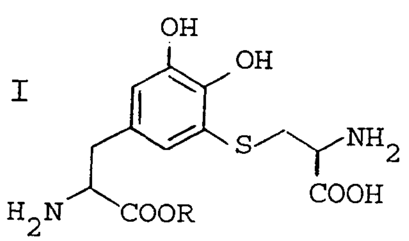

II 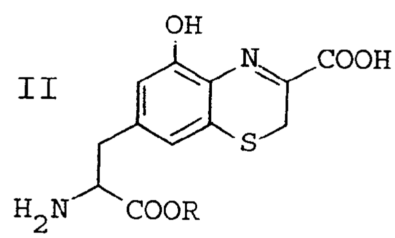

III 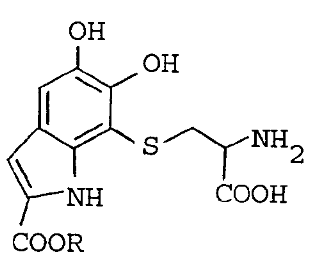

and IV 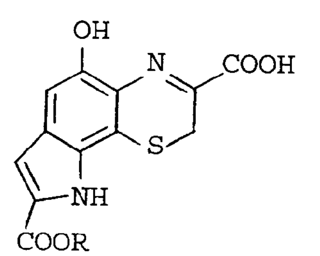

9. A composition for application to skin or hair to serve as a colourant and/or sunscreen therefor containing

as active ingredient the product of the reaction between:
(i) an alkyl tyrosine ester of the formula:

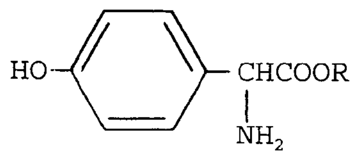

where R = $C_{1-6}$ alkyl,
(ii) tyrosinase, and optionally
(iii) cysteine.

**10.** A composition according to claim 9 wherein cysteine is present and the weight ratio of the alkyl tyrosine ester and cysteine is in the range from 1.33:1 to 2.0:1

**11.** A kit for use for colouring and/or sunscreening skin or hair comprising in a first container an alkyl ester of the formula

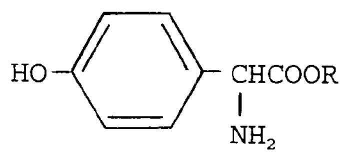

where R = $C_{1-6}$ alkyl
and in a second container, tyrosinase.

**12.** A kit according to claim 11 wherein one of the said containers also contains cysteine.

**13.** A kit according to claim 11 which comprises, in a third container, cysteine.

**14.** A composition according to claim 12 wherein the weight ratio of the alkyl tyrosine ester and cysteine is in the range from 1.33:1 to 2.0:1

**15.** A method of colouring and/or sunscreening skin or hair comprising topically applying
(i) an alkyl tyrosine ester of formula

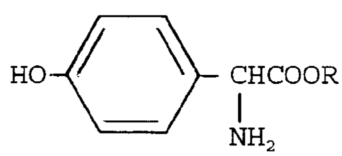

where R = $C_{1-6}$ alkyl
(ii) tyrosinase, and optionally
(iii) cysteine
or the product of the reaction between (i), (ii) and optionally (iii).

**16.** A composition for application to skin or hair to serve as a colourant and/or sunscreen therefor containing as active ingredient the product of the reaction between:
(a) an alkyl tyrosine ester of the formula:

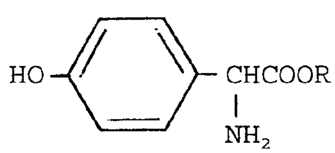

where R = $C_{1-6}$ alkyl,
together with cysteine; or

(b) a product of reaction thereof which has a formula selected from the group consisting of:

I 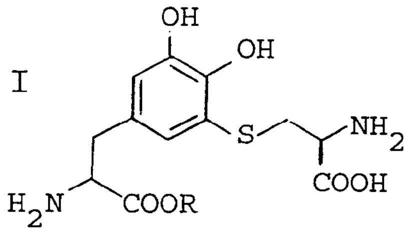

II 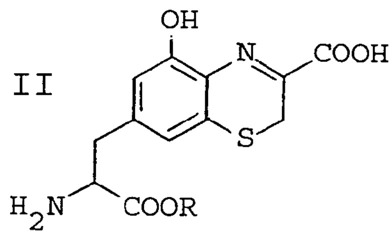

III 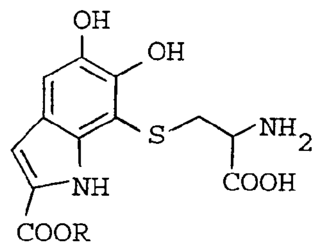

and IV 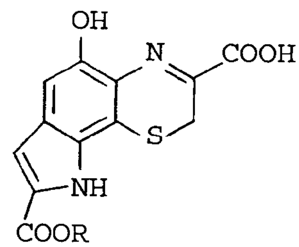

**17.** A composition according to claim 16 comprising said alkyl tyrosine ester and cysteine in a weight ratio lying in the range 1.33:1 to 2.0:1.

**18.** A composition of matter comprising a material selected from the group consisting of compounds of the formulae

I 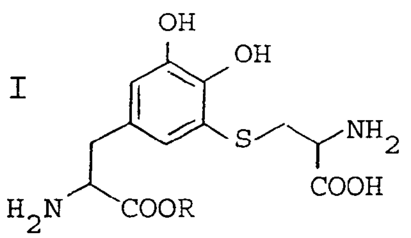

II 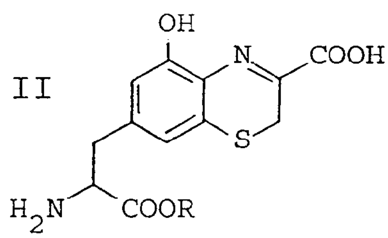

III 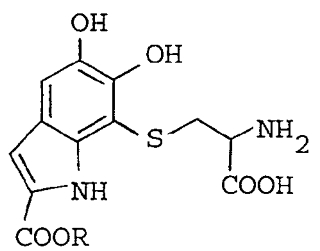

and IV 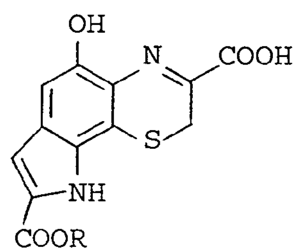

and products formed by polymerisation of one or more of the said compounds.

**19.** Use of an alkyl tyrosine ester of the formula

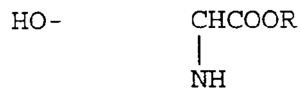

or a product obtainable therefrom by reaction with tyrosinase, as a colourant or sunscreen agent, for application to skin to colour the skin or hair growing therefrom at locations other than melanocytes.

Fig. 1.

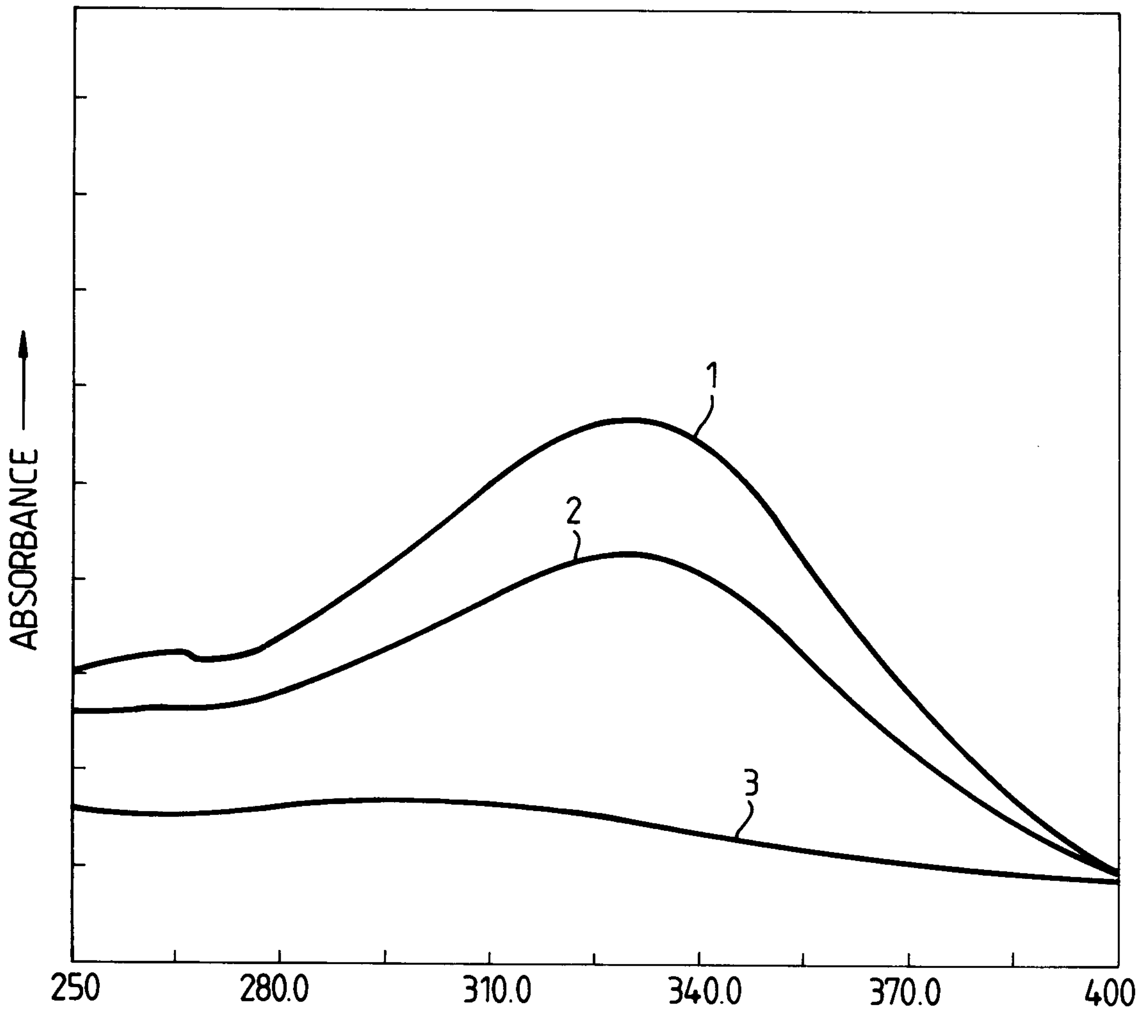


1 = PRODUCT FROM METHYL TYROSINE

2 = PRODUCT FROM ETHYL TYROSINE

3 = PRODUCT FROM TYROSINE

Fig. 2.

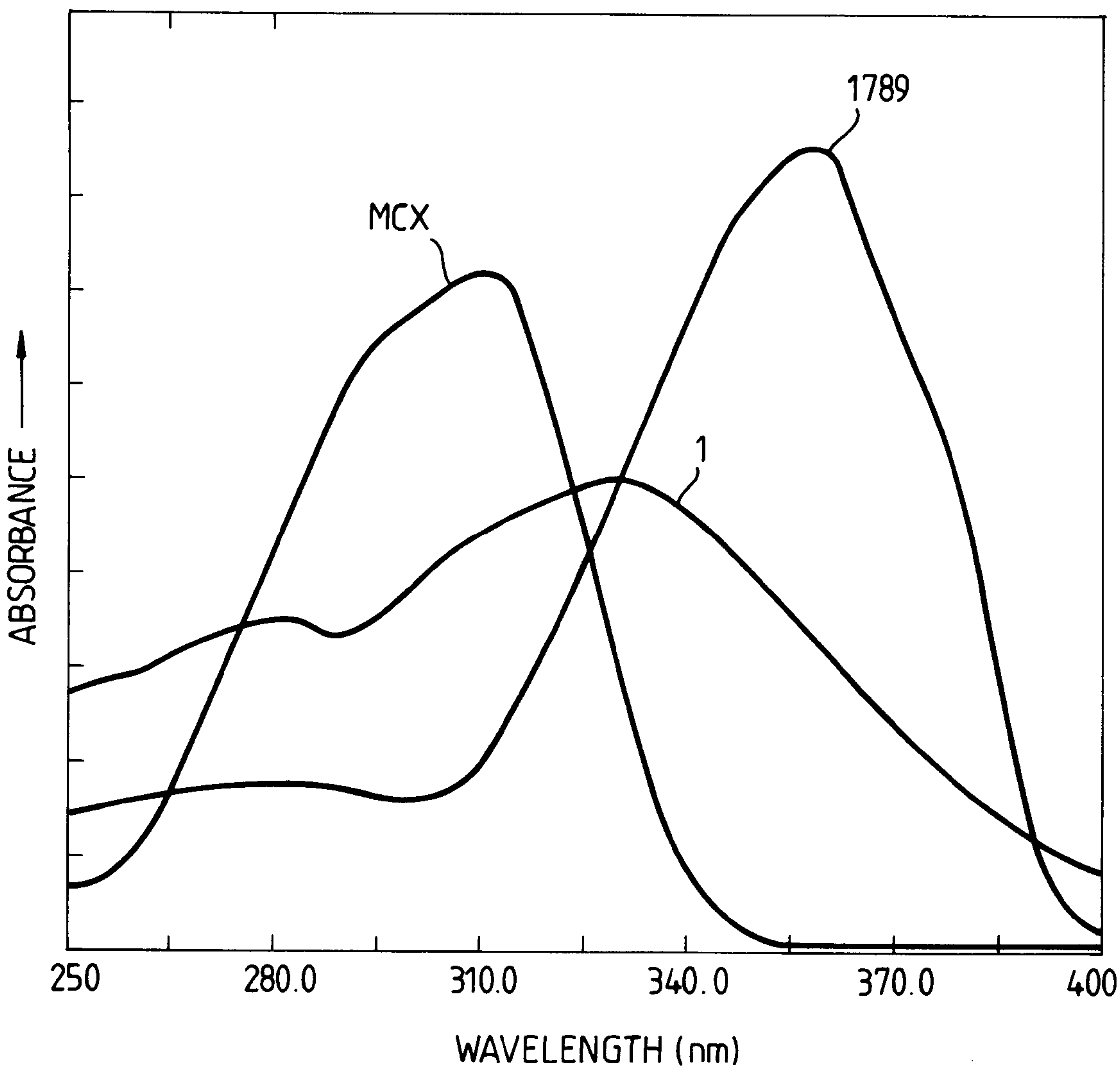


1 = PRODUCT FROM METHYL TYROSINE
MCX = PARSOL MCX
1789 = PARSOL 1789

Fig. 3.

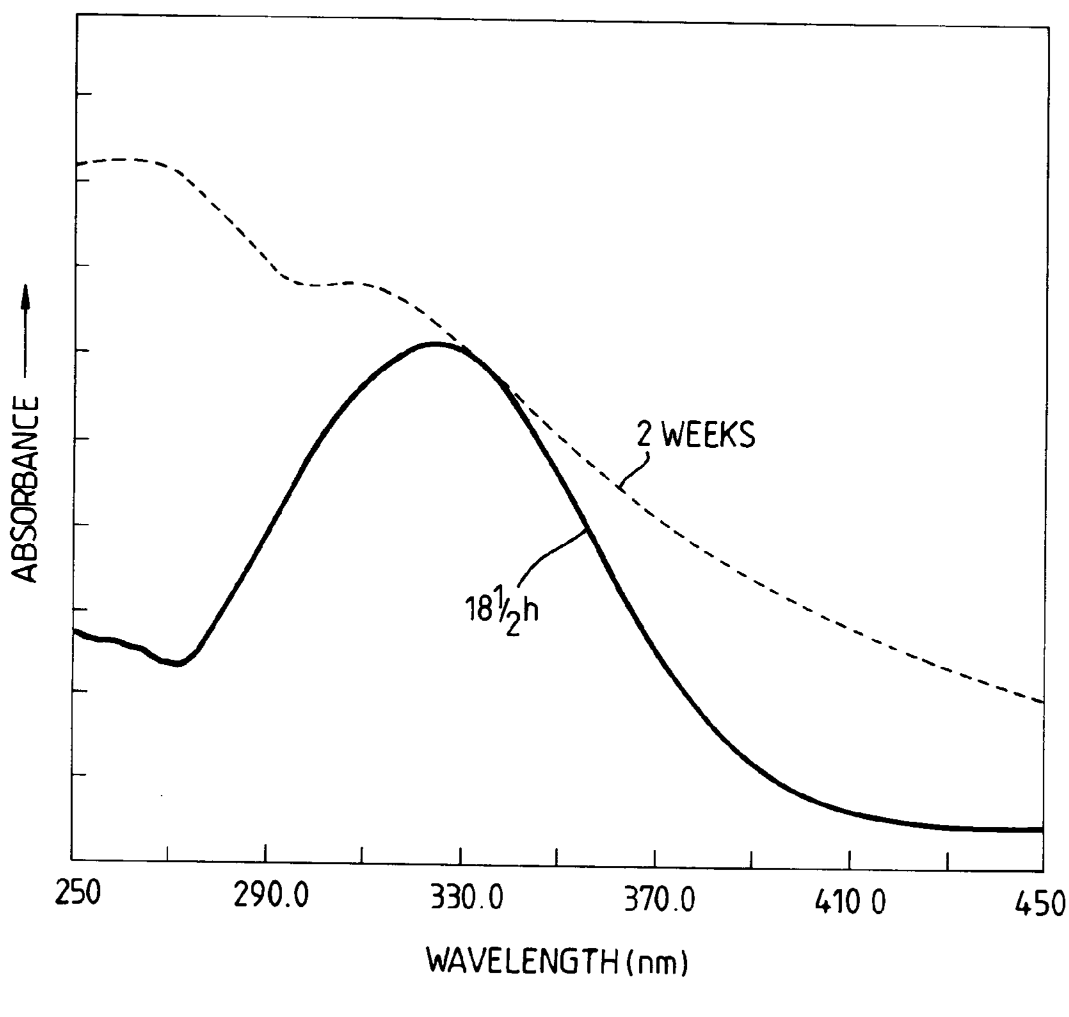
ABSORBANCE
2 WEEKS
18½h
250
290.0
330.0
370.0
410 0
450
WAVELENGTH (nm)

Fig. 4.

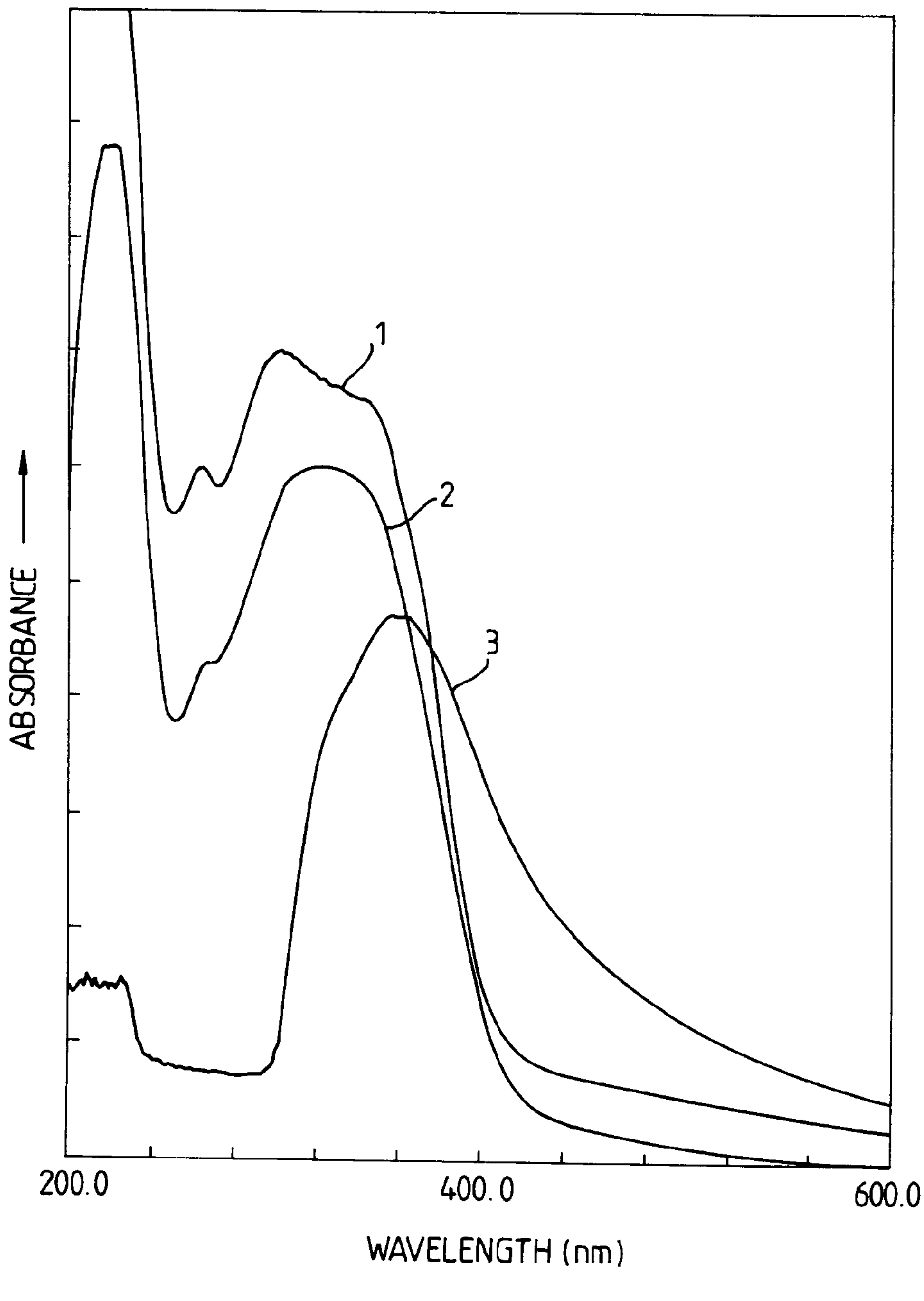


1 = PRODUCT FROM METHYL TYROSINE

2 = SAME PRODUCT CONJUGATED TO CASEIN

3 = SAME PRODUCT CONJUGATED TO KERATIN

Fig. 5.

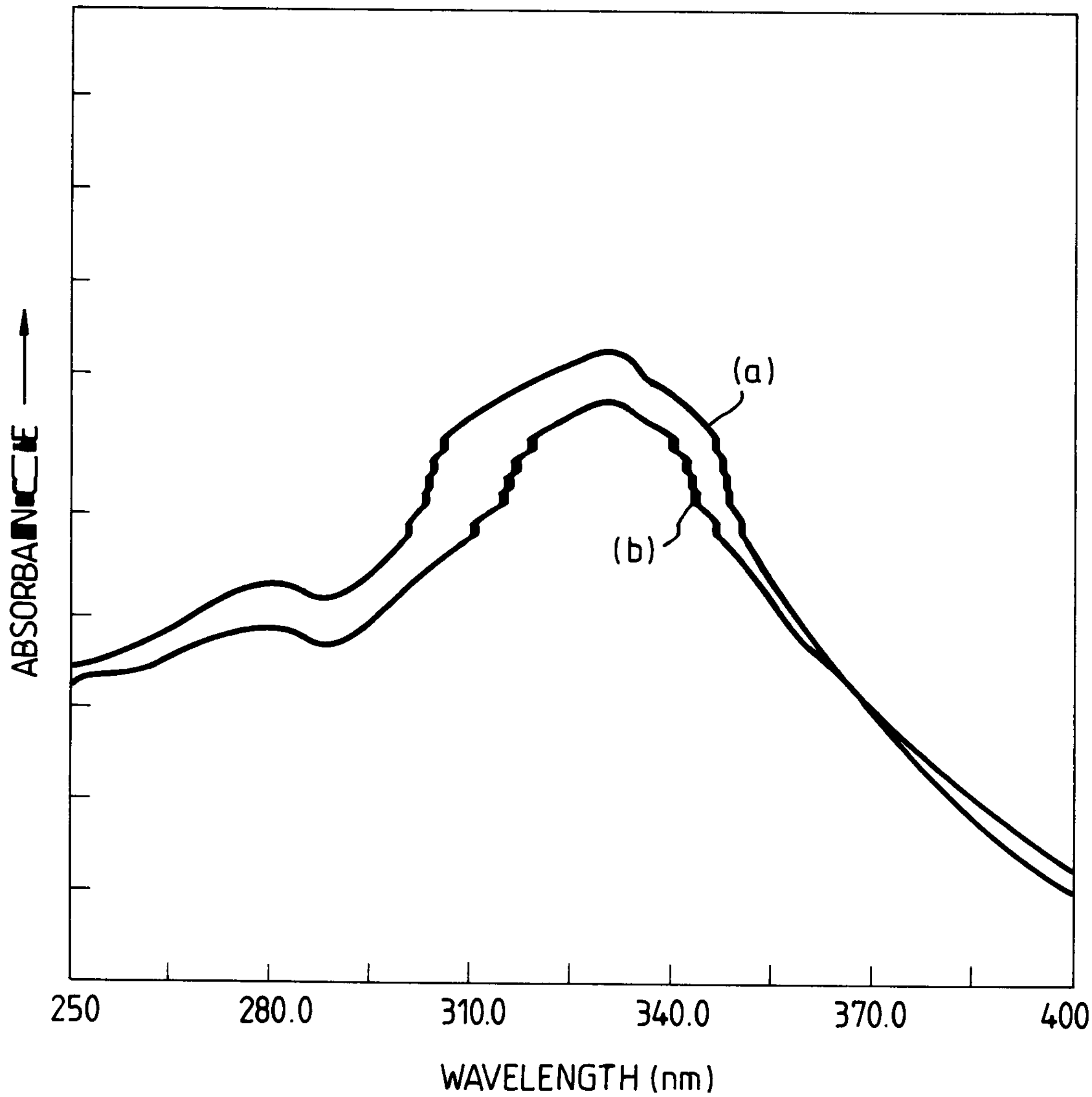

Fig. 6.

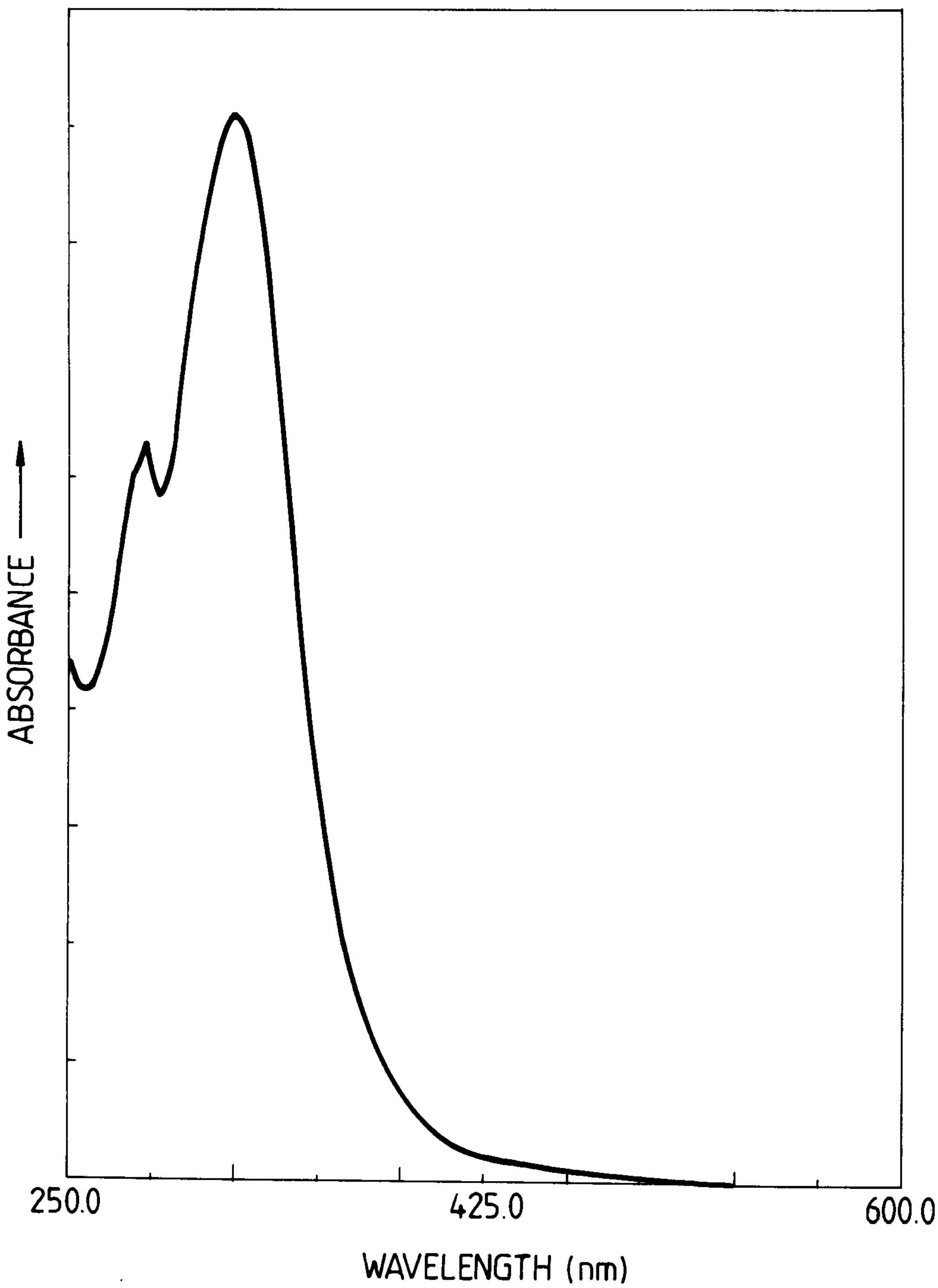

Fig. 7.

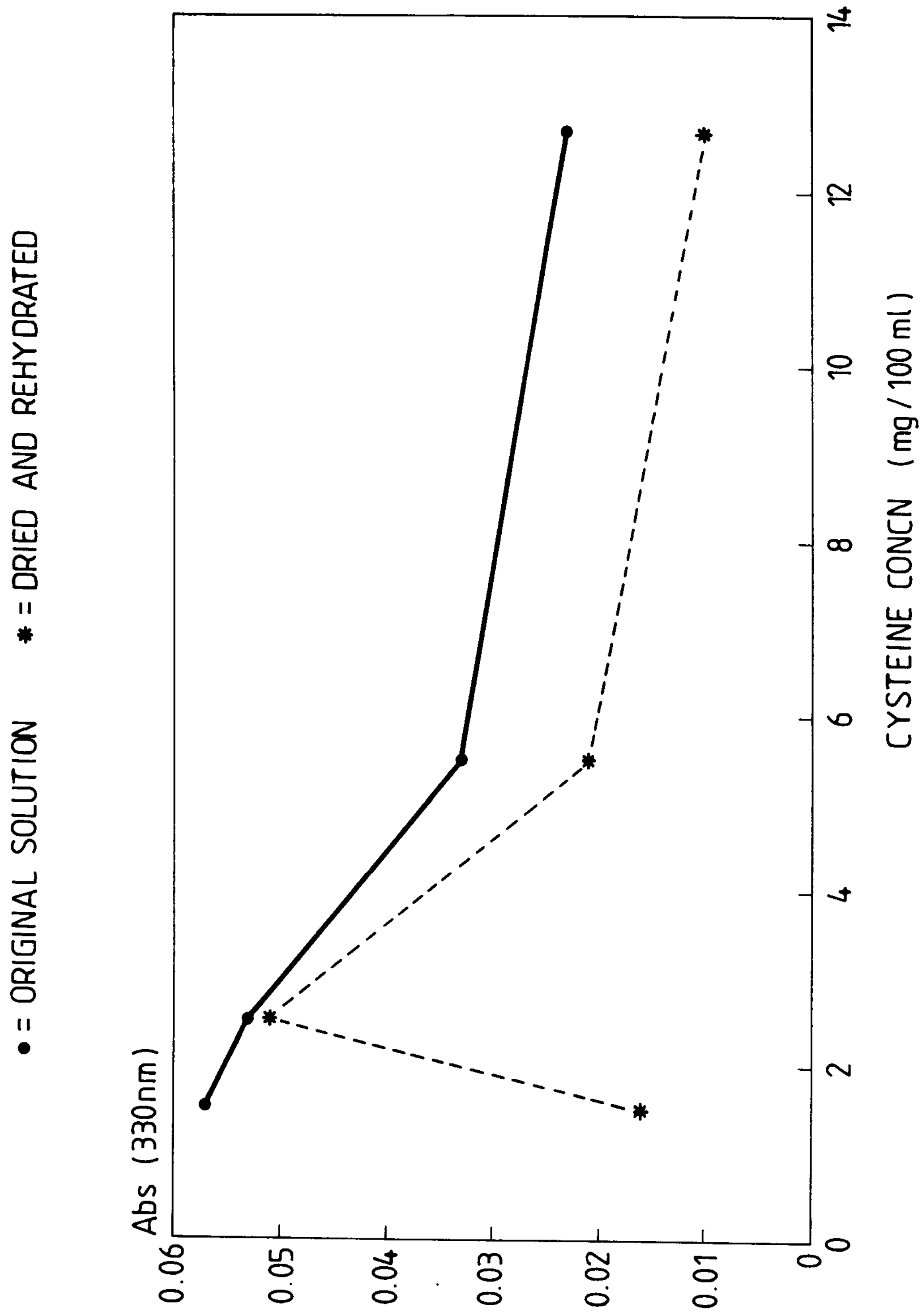
● = ORIGINAL SOLUTION
✱ = DRIED AND REHYDRATED
Abs (330nm)
0.06
0.05
0.04
0.03
0.02
0.01
0
2
4
6
8
10
12
14
CYSTEINE CONCN (mg / 100 ml)

European Patent Office

# PARTIAL EUROPEAN SEARCH REPORT

which under Rule 45 of the European Patent Convention shall be considered, for the purposes of subsequent proceedings, as the European search report

Application Number: EP 93 30 6417

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X,D | US-A-4 021 538 (R. J. YU) 3 May 1977<br>* column 1, line 1 - line 16 *<br>* column 2 * | 1,5,9,15 | A61K7/42<br>A61K7/13 |
| X | EP-A-0 356 119 (BRISTOL-MYERS COMPANY) 28 February 1990<br>* page 3, line 32 - line 38 *<br>* page 4, line 29 - line 61 *<br>* page 5, line 63 * | 1,5,6,15 | |
| Y | | 8,16,18 | |
| X | GB-A-2 197 885 (L'OREAL) 2 June 1988<br>* page 7, paragraph 2 -paragraph 3 *<br>* page 9, paragraph 5 *<br>* page 11, paragraph 5 *<br>* page 20; example 12 *<br>* page 23; example 17 * | 1,5,9,15 | |
| A | | 11 | |

-/--

TECHNICAL FIELDS SEARCHED (Int.Cl.5)

A61K

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

see sheet C

| Place of search | Date of completion of the search | Examiner |
|---|---|---|
| THE HAGUE | 25 November 1993 | Gac, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

# PARTIAL EUROPEAN SEARCH REPORT

Application Number
EP 93 30 6417

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| Y | EP-A-0 313 380 (ADVANCED POLYMER SYSTEMS, INC) 26 April 1989<br>* page 5, line 53 - line 65 *<br>* page 6, line 6 - line 32 *<br>* page 7, line 48 - line 65 *<br>* page 8, line 1 - line 10 *<br>* page 8, line 64 *<br>--- | 1,5,6,8, 9,15,16, 18 | |
| Y | FR-A-2 252 841 (SHISEIDO CO LTD) 27 June 1975<br>* the whole document *<br>--- | 1,5,6,9, 11-13,15 | |
| Y | FEBS LETTERS<br>vol. 125, no. 1 , 1981<br>pages 101 - 103<br>NOVELLINO ET AL. 'Identification of cysteinyldopa-derived units in eumelanins from mammalian eyes'<br>* the whole document *<br>--- | 1,2,7-9, 15,16, 18,19 | TECHNICAL FIELDS SEARCHED (Int.Cl.5) |
| Y | ACTA DERMATO-VENEREOLOGY<br>vol. 63, no. 6 , 1983<br>pages 463 - 467<br>ITO ET AL. 'Protein-bound DOPA and 5-S-Cysteinyldopa in non-melanogenic tissues'<br>* the whole document *<br>--- | 1,2,7-9, 15,16, 18,19 | |
| Y | US-A-4 946 472 (M. MOTONO) 7 August 1990<br>* the whole document * | 11-13 | |
| A | | 2-6 | |
| | ---<br>-/-- | | |

European Patent Office

# PARTIAL EUROPEAN SEARCH REPORT

Application Number
EP 93 30 6417

| DOCUMENTS CONSIDERED TO BE RELEVANT | | | CLASSIFICATION OF THE APPLICATION (Int.Cl.5) |
|---|---|---|---|
| Category | Citation of document with indication, where appropriate, of relevant passages | Relevant to claim | |
| A | TETRAHEDRON<br>vol. 43, no. 22 , 1987<br>pages 5357 - 5362<br>D'ISCHIA ET AL. 'Sulphydryl compounds in melanogenesis. Part II. reactions of cysteine and glutathione with dopachrome'<br>* the whole document *<br>--- | 1,7-9, 16,18 | |
| A | COSMETICS & TOILETRIES / SUN PRODUCTS DOCUMENTARY / FORMULARY / ENCYCLOPEDIA<br>vol. 98 , March 1983<br>pages 47 - 50<br>M.M RIEGER ET AL. 'Current aspects of cosmetic science : VIII : the chemistry of tanning'<br>--- | 1,7-9, 15,16, 18,19 | TECHNICAL FIELDS SEARCHED (Int.Cl.5) |
| A | EP-A-0 010 483 (LABORATOIRES SEROBIOLOGIQUES S.A.) 30 April 1980<br>* page 5 *<br>* page 6, line 28 - line 32 *<br>* page 12, line 22 *<br>* page 13, line 25 *<br>--- | 1,3-6,9, 15,16 | |
| A | CH-A-642 537 (UNI-CHEMIE AG) 30 April 1984<br>* the whole document *<br>----- | 1,5,9,15 | |

EPO FORM 1503 03.82 (P04C10)

EP 93 30 6417 -C-

The structure of tyrosine has an additional "$-CH_2-$" group between the phenyl and the aminoacidic groups (see Merck Index 11th Edition 1989, page 1548, no. 9747).
Since the reaction products as defined in claim 8 and in page 5, lines 15-21 of the description do also possess this methylene group in their formulas, its appears unlikely that the structure of the so-called "tyrosine esters" of claims 1(i),9,11,15,16, 19 is the correct one.
The search was performed understanding the term "tyrosine" as described in the Merck Index.